# EUROPEAN PATENT APPLICATION

(11) **EP 3 494 935 A1**
(43) Date of publication of application: **12.06.2019**
(21) Application number: 18198462.6
(22) Date of filing: 03.10.2018
(51) Int. Cl.: A61F 2/95

(54) **STENT DELIVERY BALLOON CATHETER ASSEMBLY**

(30) Priority: 03.10.2017 US 201762567482 P
(71) Applicant: Cook Medical Technologies LLC, Bloomington, IN 47404 (US)
(72) Inventor: EATON, Elizabeth, Bloomington, IN 47404 (US)
(74) Representative: Leach, Sean Adam

(57) **Abstract**

A stent delivery balloon catheter assembly includes a catheter that defines exactly one or two lumens, which includes an inflation lumen that extends from an inflation port at a proximal end to open through a distal end of the catheter. The catheter defines a wire guide channel that terminates at a wire guide exit located a distance from the distal end of the catheter. A balloon with a closed distal end is attached to the catheter distally of the wire guide exit, and extends distally beyond the distal end of the catheter. The balloon is in fluid communication with the inflation lumen. A column strength stiffener has a proximal end attached to the catheter distally of the wire guide exit, and has a balloon support segment extending distally away from the distal end of the catheter within the balloon. A distal end of the column strength stiffener is attached to the closed distal end of the balloon.

## Description

### Technical Field

The present disclosure relates generally to balloon expanded stent delivery systems, and more particularly to profile reduction with a stent delivery balloon catheter assembly that includes a tipless balloon with a closed distal end.

### Background

One of the challenges of balloon expandable covered stents is delivery profile. Covered stenting of visceral vessels coincident with fenestrated endograft implantation in individuals with Crawford II, III, or IV (as well as infrarenal with insufficient proximal neck length) aortic aneurysms is an essential aspect of treatment insuring continued blood flow to critical organs and systems while fully excluding the aneurysm sac. In the United States market alone, few options are available for the preferred (balloon-expandable) stents, and it remains widely accepted that there is room for improvement in the technology.

In order to reduce operating time, requisite operator skill level, imaging requirements, as well as to expand the patient demographic eligible for endovascular repair, the industry and clinicians are moving increasingly towards pre-loaded, pre-cannulated, lower profile endograft delivery systems. This means the profile requirements for the accessory products (including balloon expanded covered stent delivery systems) are shrinking. It is within this context that the importance of reducing balloon catheter profiles is understood for this disclosure.

The present disclosure is directed toward one or more of the problems set forth above.

### Summary of the Disclosure

In one aspect, a stent delivery balloon catheter assembly includes a catheter that defines exactly one or two lumens, which includes an inflation lumen that extends from an inflation port at a proximal end to open through a distal end of the catheter. The catheter defines a wire guide channel that terminates at a wire guide exit located a distance from the distal end of the catheter. A balloon with a closed distal end is attached to the catheter distally of the wire guide exit, and extends distally beyond the distal end of the catheter. The balloon is in fluid communication with the inflation lumen. A column strength stiffener has a proximal end attached to the catheter distally of the wire guide exit, and a balloon support segment extending distally away from the distal end of the catheter within the balloon. A distal end of the column strength stiffener is attached to the closed distal end of the balloon.

In another aspect, a stent delivery system includes a stent delivery balloon catheter, a sheath, a wire guide and a stent. The system has a first configuration characterized by the stent being crimped about the balloon, which is deflated and positioned inside the sheath. A distal end of the wire guide is positioned at, or proximally displaced from, the wire guide exit and within the wire guide channel. The system has a second configuration characterized by the stent and the balloon being outside of the sheath, and the distal end of the wire guide is positioned between an outer surface of the balloon, which is deflated, and an interior surface of the stent, which is expanded. A segment of the wire guide is positioned in the wire guide channel.

In still another aspect, a method of operating a stent delivery system includes moving a distal end of a sheath from within a fenestrated stent graft and out through a fenestration of the fenestrated stent graft. A stent delivery balloon catheter is moved through the sheath to a position at which the balloon of the stent delivery balloon catheter is within the sheath, and most or all of the length of the balloon is outside of the fenestrated stent graft. A wire guide is positioned in a wire guide channel of the delivery balloon catheter so that a distal end of the wire guide is positioned at, or proximately displaced from, a wire guide exit located proximal to the balloon. The sheath is moved in a proximal direction to uncover a covered stent graft that is crimped about the balloon, while maintaining a position of the stent delivery balloon catheter. The covered stent graft is expanded by inflating the balloon, which is then deflated. The distal end of the wire guide is advanced out through the wire guide exit, along an outer surface of the balloon, into the expanded covered stent graft and out through a distal end of the covered stent graft. The stent delivery balloon catheter is withdrawn in a proximal direction along the wire guide, while maintaining the wire guide in place.

### Brief Description of the Drawings

Fig. 1 is a side schematic view of a stent delivery balloon catheter assembly according to the present disclosure;
Fig. 2 is a sectioned view of the catheter of Fig. 1 as viewed along section lines 2-2;
Fig. 3 is a sectioned view similar to Fig. 2, except showing an alternative construction according to the present disclosure;
Fig. 4 is a schematic side view of a stent delivery system according to the present disclosure in a first configuration;
Fig. 5 is a side schematic view of the stent delivery system of Fig. 4 shown in a second configuration;
Fig. 6 is a side schematic view of the stent delivery system of Figs. 4 and 5 shown in a third configuration;
Fig. 7 is a schematic view of a moment during an aortic aneurysm repair procedure according to an aspect of the present disclosure;
Fig. 8 is a later step in the aortic aneurysm repair according to the present disclosure;
Fig. 9 is a schematic view of the aortic aneurysm repair after the stent delivery system has been positioned in the first configuration in one of the renal arteries;
Fig. 10 is a schematic side view of a later step in the aortic aneurysm repair procedure according to the present disclosure;
Fig. 11 is a later step in the aortic aneurysm repair procedure with the balloon inflated to expand a covered stent in the renal artery;
Fig. 12 is a schematic view of the aortic aneurysm repair after the balloon has been deflated;
Fig. 13 is a schematic view of the aortic aneurysm repair procedure with the stent delivery system shown in its second configuration;
Fig. 14 is a schematic view of the aortic aneurysm repair procedure with the stent delivery system shown in the third configuration;
Fig. 15 is a schematic view of the aortic aneurysm repair procedure after the stent delivery balloon catheter is being withdrawn;
Fig. 16 is a schematic view of the aortic aneurysm repair procedure with an optional catheter being advanced along the wire guide toward the renal artery; and
Fig. 17 is a schematic view after the aortic aneurysm repair procedure has been completed according to the present disclosure.

### Detailed Description

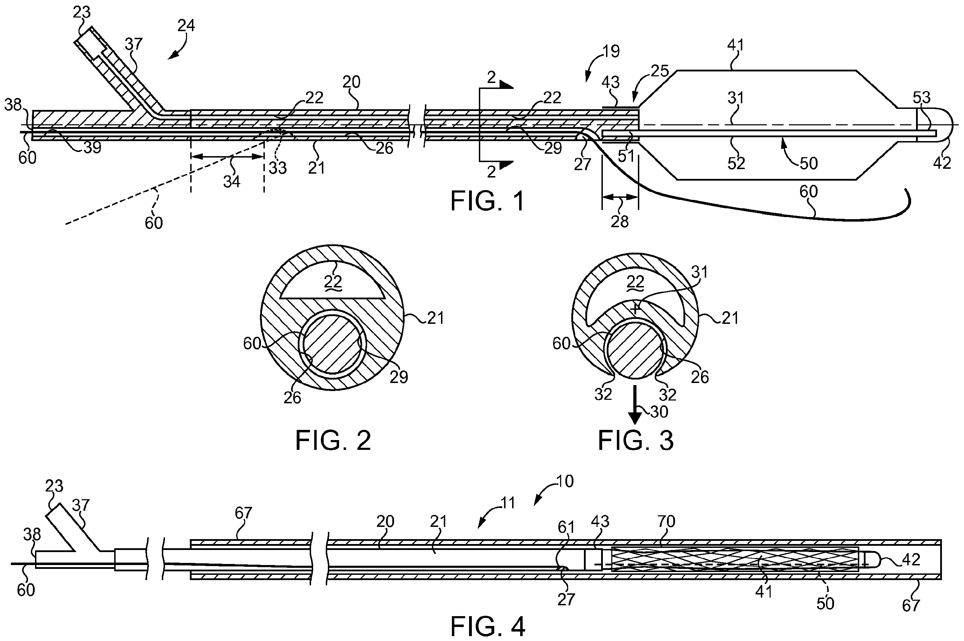

Referring initially to figures 1 and 2, a stent delivery balloon catheter assembly 19 includes a balloon catheter 20, which includes a catheter 21 that defines exactly one or two lumens. In all embodiments, an inflation lumen 22 that extends from an inflation port 23 at a proximal end 24 to open through a distal end 25 of the catheter 21 is one of the exactly one or two lumens. Catheter 21 also defines a wire guide channel 26 that terminates at a wire guide exit 27 located a distance 28 from the distal end 25 of catheter 21. A balloon 41, which has a closed distal end 42, is attached to the catheter 21 distally of the wire guide exit 27. Balloon 41 extends distally beyond the distal end 25 of catheter 21, and the balloon 41 is in fluid communication with the inflation lumen 22. A column strength stiffener 50 has a proximal end 51 attached to the catheter 21 distally of the wire guide exit 27. In one specific example, column strength stiffener 50 may be on the order of 0.035 inches in diameter, made from a suitable medical grade metallic material, and may be heat weld sealed into the catheter 21 where a wire guide lumen might otherwise be located. A balloon support segment 52 of the column strength stiffener 50 extends distally away from the distal end of catheter 21 within the balloon 41. A distal end 53 of the column strength stiffener 50 is attached, such as by a second heat weld, to the closed distal end 42 of the balloon 41.

As shown in the section view Fig. 2, the wire guide channel 26 may be a wire guide lumen 29 of the exactly two lumens. Fig. 3 shows an alternative embodiment in which the catheter 21 defines exactly one lumen, which is the inflation lumen 22. In the Fig. 3 variant, wire guide channel 26 opens radially outward in a direction 30 away from a center line 31 of catheter 21 between two channel edges 32 that are out of contact with each other by a distance that is less than a diameter of the wire guide 60. In the alternative figure 3 embodiment, the catheter 21 may achieve a smaller profile than the counterpart cross-section shown in Fig. 2. In Fig. 3, some profile shrinkage is gained by shaping the inflation lumen to have a nested shape and by shaping the wire guide channel 26 to be something other than an enclosed lumen as in the Fig. 2 embodiment. The Fig. 2 embodiment includes a circular shaped wire guide lumen 29 and a D-shaped inflation lumen 22. Nevertheless, those skilled in the art will appreciate that other shapes and strategies may be utilized without departing from the present disclosure. Those skilled in the art will appreciate that the largest profile portion of assembly 19 may normally occur in the vicinity of balloon 41 when a balloon expandable stent is crimped about the balloon. The present disclosure teaches the achievement of a smaller profile by crimping a stent about balloon 41, which is supported by relatively low profile column strength stiffener 50 instead of a segment of catheter as in the prior art.

Stent delivery balloon catheter assembly 19 may include a hub fitting 37 attached to the proximal end 24 of catheter 21. The hub fitting 37 may include the inflation port 23 and a wire guide port 38, which may be considered as a proximal end 39 of wire guide channel 26. In an alternative strategy, a proximal end of the wire guide channel 26 may be facilitated at a wire guide entrance 33 that is located a distance 34 along catheter centerline 31 from the proximal end 25 of the catheter. Wire guide entrance 33 may take a shape and form similar to wire guide exit 27, where a portion of the outer wall of the catheter is cut away or removed to allow ingress and egress with a wire guide 60 in through wire guide entrance 33 and out through wire guide exit 27. Fig. 1 shows a wire guide 60 extending from wire guide port 38 and out through wire guide exit 27, respectively. The alternative version shows a proximal segment of a wire guide 60 in dashed lines extending from wire guide entrance 33. The alternative strategy allows the wire guide to not have to pass through hub 37. Although not necessary, because practitioners often find preference with the use of wire guides having a diameter on the order of 0.035 inches, the wire guide channel 26 may preferably by sized to slidably receive a 0.035 inch diameter wire guide. Also in one specific example, the wire guide exit 27 may be at least one centimeter away from a proximal balloon bond 43 to catheter 21.

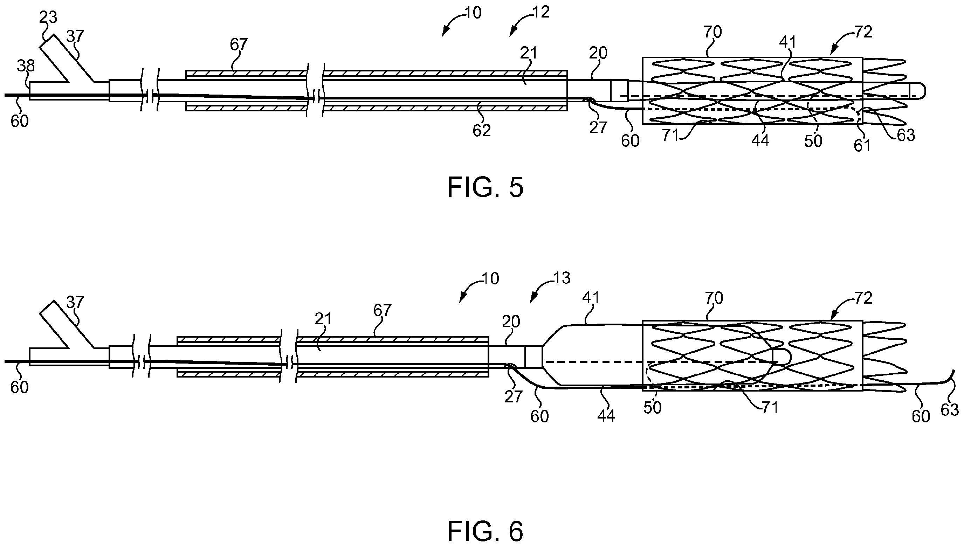

Referring now in addition to Figs. 4-6, a stent delivery system 10 includes a stent delivery balloon catheter 20, a stent 70, a sheath 67, and a wire guide 60. Fig. 4 shows stent delivery system 10 in a first configuration 11 that is characterized by the stent 70 being crimped about the balloon 41, which is deflated and positioned inside the sheath 67. A distal end 61 of the wire guide 60 is positioned at, or proximally displaced from, the wire guide exit 27 and within the wire guide channel 26. This arrangement leaves the distal end 61 of the wire guide 60 out of contact with sheath 67 in the first configuration. As shown in Fig. 5, a stent delivery system 10 has a second configuration 12 that is characterized by the stent 70 and the balloon 41 being outside of the sheath 67, such as beyond the distal end of the sheath 67 as shown. The distal end 61 of wire guide 60 is positioned between an outer surface 44 of the balloon 41, which is deflated, and an interior surface 71 of the stent 70, which is expanded. A segment 62 of the wire guide remains positioned in the wire guide channel 26. Although the present disclosure encompasses plain uncovered stents, stent 70 is preferably a covered stent graft 72 having a structure well-known in the art in which a framework, usually of metallic struts, supports a tube constructed of a fabric and/or plastic sheeting in a manner well-known in the art. Although not necessary, wire guide 60 may have a curved distal segment 63, such as a J-shape, and may have a diameter of 0.035 inches in accordance with the preferred diameter of many practitioners. Nevertheless, other wire guide shapes and diameters would also fall within the intended scope of the present disclosure. Fig. 6 shows that the stent delivery system 10 may also include a third configuration 13 that is characterized by the wire guide 60 being pinched between the outer surface 44 of the balloon 41, which is inflated, and less than a full length of the interior surface 71 of stent 70. This configuration may be utilized to better fully open one end of stent 70 and/or to deploy flared ends such as in the case of being joined to a fenestrated stent graft, as described infra. In other words, balloon 41 may be positioned in a fenestration (not shown) of a fenestrated stent graft (not shown) in the third configuration 13 of Fig. 6.

Those skilled in the art will appreciate that all the components of stent delivery system 10 in general, and the stent delivery balloon catheter 20 in particular, may be constructed from known materials used in like medical devices, and no exotic materials or techniques are necessary in order to make and use the device(s) of the present disclosure.

### Industrial Applicability

The present disclosure finds general applicability to any balloon expanded stent application, especially where lower profiles may provide an advantage. The present disclosure finds particular applicability in the delivery of covered stent grafts. Finally, the present disclosure may find a preferred application in the delivery of covered stent grafts to be joined to a fenestrated stent graft, such as for supporting openings to the renal arteries when treating an aortic aneurysm by bridging the aneurysm with covered stent grafts.

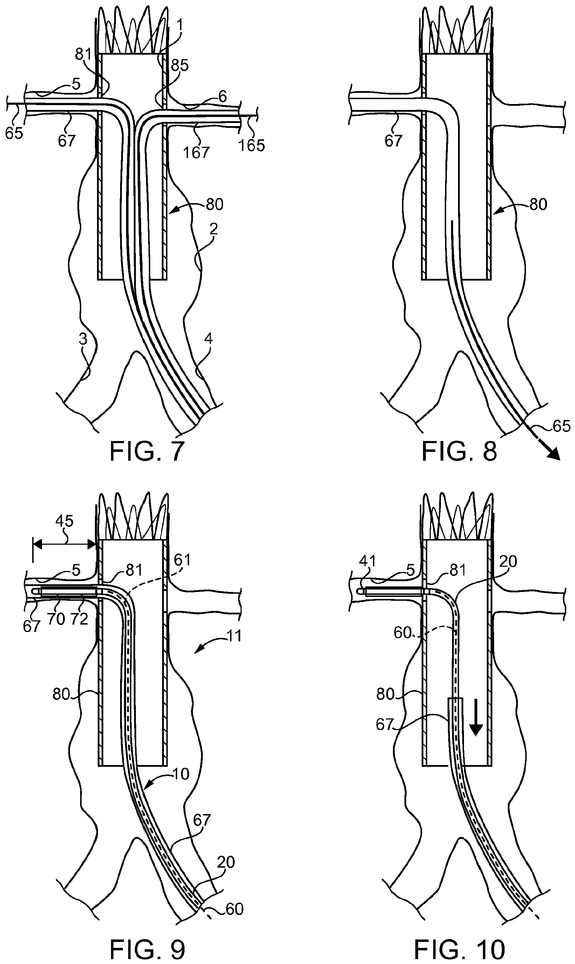

Referring now to Figs. 7-17, the stent delivery system 10 of the present disclosure is shown being used to support the openings to the renal arteries 5 and 6 as part of an overall aortic aneurysm repair utilizing a fenestrated stent graft 80 that includes fenestrations 81 and 85 aligned with the respective renal arteries 5 and 6. Thus, the fenestrated stent graft 80 shown in Fig. 7 has already been delivered to the aorta 1 in a conventional manner and partially bridges the aneurysm 2, which is located just upstream from the iliac arteries 3 and 4. After positioning wire guides 65 and 165 to extend through fenestrated stent graft 80 and out into the respective renal arteries 5 and 6, sheaths 67 and 167 may be slid in a distal direction into the positions shown in Fig. 7 along the respective wire guides 65 and 165. The sheath 67 may be positioned by moving a distal end of the sheath from within the fenestrated stent graft 80 and out through the fenestration 81 as guided by wire guide 65. Thereafter, the sheath 67 may be left in place and the respective wire guide 65 withdrawn from the sheath 67 as shown in Fig. 8. The procedure described infra and in relation to Figs. 8-16 only refer to the renal artery 5 as the procedure is duplicated with regard to the renal artery 6. Also, the illustrations of Figs. 8-16 are simplified by omitting the sheath 167 from these figures. After withdrawing wire guide 65, the stent delivery balloon catheter 20 is advanced through the sheath 67 until the stent delivery system 10 assumes the first configuration 11 as shown in Fig. 9 and Fig. 4. The stent delivery system 10 may arrive at the first configuration 11 by allowing the stent delivery balloon catheter 20 and the wire guide 60 to be advanced simultaneously through the sheath 67. In such a case, the wire guide exit 27 may be positioned no less than several centimeters in the proximal direction from the proximal balloon bond 43, to allow the stent 70 and balloon 41 to pass fully through the sheath hemostatic valve (not shown) before the wire 60 is introduced. Alternatively, the stent delivery balloon catheter 20 may be slid through sheath 67 and positioned as shown in Fig. 9, and thereafter the wire guide 60 may be advanced along the wire guide channel 26 of the stent delivery balloon catheter 20 until the distal end 61 of the wire guide 60 is at or just proximal to the wire guide exit 27. It should be noted that in the first configuration 11, the covered stent 72 is located approximately where it will be deployed, but is still covered by sheath 67. Thus, in order to arrive at first configuration 11 shown in Fig. 9, the stent delivery balloon catheter 20 is moved through the sheath 67 simultaneously with the wire guide, or prior to the positioning of the wire guide 60 to a point at which the balloon 41 is within sheath 67, and most or all of the length 45 of the balloon 41 is outside of the fenestrated stent graft 80.

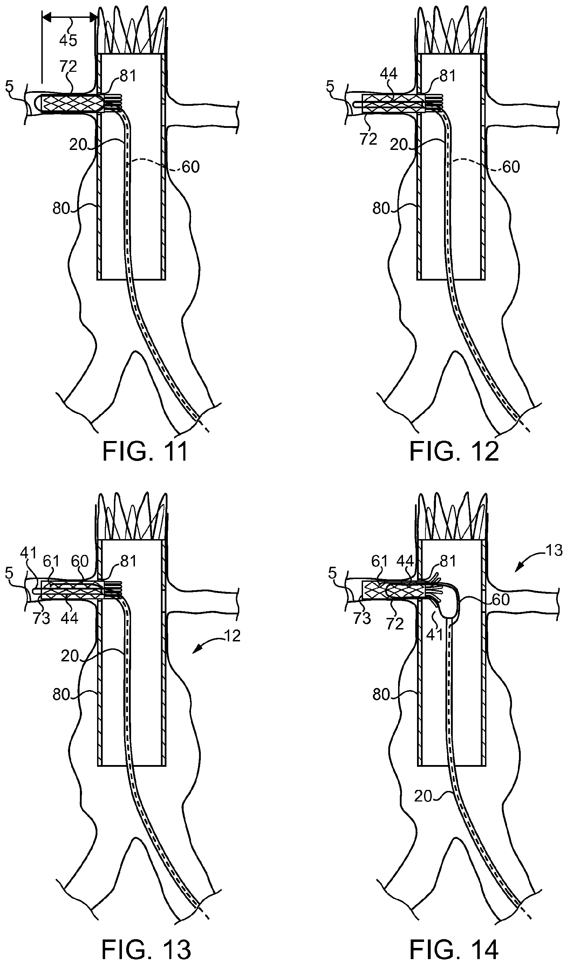

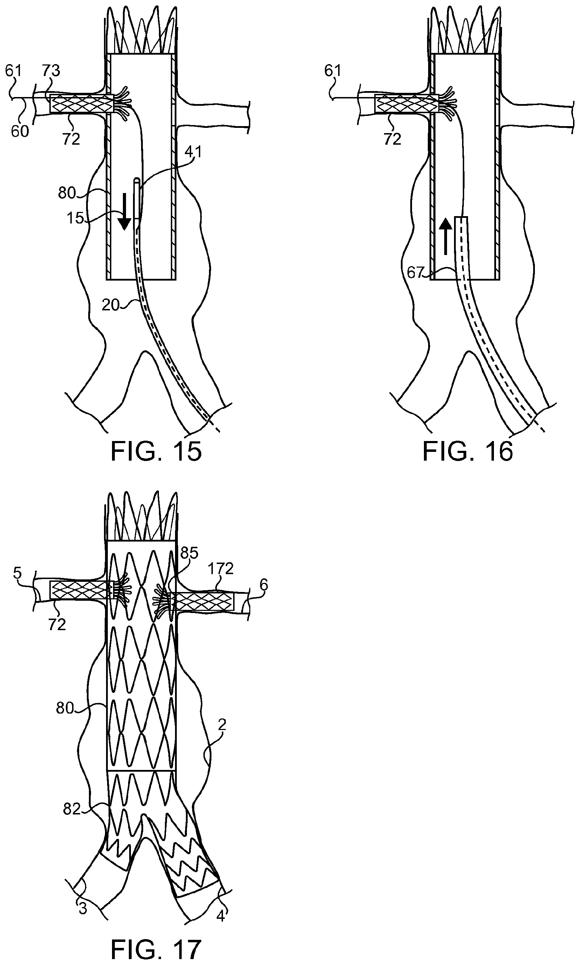

After achieving the first configuration 11 of Fig. 9, the sheath 67 may be moved in a proximal direction to uncover the stent graft 72, which is crimped about balloon 41, while maintaining a position of the stent delivery balloon catheter 20 as shown in Fig. 10. Next, as shown in Fig. 11, the covered stent graft 72 is expanded within the renal arterys and the fenestration 81 by inflating the balloon 41. Next, the balloon is deflated as shown in Fig. 12. Thereafter, the previously concealed wire guide 60 may be advanced such that the distal end 61 of the wire guide 60 is moved out through the wire guide exit 27, along an outer surface 44 of the balloon 41, and into the expanded covered stent graft 72 to achieve the second configuration 12 shown in Fig. 13. Thereafter, the wire guide 60 may be further advanced out through a distal end 73 of the covered stent graft 72 at some later point in the procedure. Optionally the stent delivery balloon catheter 20 may be withdrawn in a proximal direction slightly in order to better connect the stent graft 72 to the fenestrated stent graft 80 at fenestration 81 by positioning balloon 44 partially within covered stent graft 72 and partially within fenestrated stent graft 80, but within fenestration 81 to achieve the third configuration 13 as shown in Fig. 14. Thereafter, or if the step shown in Fig. 14 is omitted, the stent delivery balloon catheter 20 is withdrawn in a proximal direction 15 along wire guide 60, while maintaining the wire guide 60 in place as shown in Fig. 15. Thereafter, as optionally shown in Fig. 16, the sheath 67 or a different sheath may be re-advanced along wire guide 60 in order to facilitate insertion of possible additional accessory devices including but not limited to a self expanding stent, or additional balloons, over the wire guide 60 as is normal to complete the stenting procedure. The same procedure as previously described may then be carried with regard to second renal artery 6 using a second covered stent graft 172 extending into contact with fenestration 85 of fenestrated stent graft 80. The aortic aneurysm repair may then be completed in a known manner by positioning a branched covered stent 82 in a manner well known in the art to complete the bridging of aneurysm 2.

As stated previously, the wire guide 60 may be advanced through sheath 67 simultaneously with stent delivery balloon catheter 20, or after the stent balloon delivery catheter 20 is positioned at its desired deployment location, or any combination between those two extremes. Although not shown, the present disclosure also contemplates a scenario in which the wire guide 65 shown in Fig. 7 remains in place and the stent delivery balloon catheter 20 is passed over the wire guide 65 while being advanced up into the position shown in Fig. 9 along the previously placed wire guide 60, without departing from the present disclosure.

The advantages of the above described design may allow physicians to use 0.035 inch diameter wires in their endovascular grafting cases in lieu of the less preferred 0.018 wires. Current covered stenting technology may lean toward smaller wire diameters to reduce the overall profile of the device. By crimping the covered stent 72 about the balloon 41, which is supported by the column strength stiffener 50, lower profiles can be achieved, and can optionally lead to the elimination of the wire guide during the actual stenting procedure, and instead relying upon a sheath for proper positioning of the covered stent 72 prior to deployment.

In an embodiment there is provided a stent delivery balloon catheter assembly, which includes a catheter that defines exactly one or two lumens, which includes an inflation lumen that extends from an inflation port at a proximal end to open through a distal end of the catheter. The catheter may define a wire guide channel that terminates at a wire guide exit located a distance from the distal end of the catheter. A balloon with a closed distal end may be attached to the catheter distally of the wire guide exit, and extends distally beyond the distal end of the catheter. The balloon is in fluid communication with the inflation lumen. A column strength stiffener has a proximal end attached to the catheter distally of the wire guide exit, and has a balloon support segment extending distally away from the distal end of the catheter within the balloon. A distal end of the column strength stiffener is attached to the closed distal end of the balloon.

An embodiment also provides a stent delivery system comprising: a sheath; a wire guide; a stent; and a stent delivery balloon catheter according to any one described or claimed herein, such as the stent delivery balloon catheter defined in any one of claims 1 to 8. To the extent that certain methods described herein may be applied to the living human or animal body, it will be appreciated that such methods may be non-surgical and non-therapeutic. For example, such methods may be applied ex vivo, to tissue samples that are not part of the living human or animal body. For example, the methods described herein may be practiced on meat, tissue samples, cadavers, and other non-living objects. For example, they may be used on cadavers for surgical training, such as that carried out by medical professionals in training.

The present description is for illustrative purposes only, and should not be construed to narrow the breadth of the present disclosure in any way. Thus, those skilled in the art will appreciate that various modification might be made to the presently disclosed embodiments without departing from the full and fair scope and spirit of the present disclosure. Other aspects, features and advantages will be apparent upon an examination of the attached drawings and appended claims.

### LIST OF ELEMENTS

### TITLE: STENT DELIVERY BALLOON CATHETER ASSEMBLY AND METHOD OF OPERATING SAME

### FILE: PA-8059-PRV

- 1.: aorta
- 2.: aneurysm
- 3.: illiac artery
- 4.: illiac artery
- 5.: renal artery
- 6.: renal artery
- 10.: stent delivery system
- 11.: first configuration
- 12.: second configuration
- 13.: third configuration
- 15.: proximal direction
- 19.: stent delivery balloon catheter assembly
- 20.: stent delivery balloon catheter
- 21.: catheter
- 22.: inflation lumen
- 23.: inflation port
- 24.: proximal end
- 25.: distal end
- 26.: wire guide channel
- 27.: wire guide exit
- 28.: distance
- 29.: wire guide lumen
- 30.: direction
- 31.: centerline
- 32.: channel edges
- 33.: wire guide entrance
- 34.: distance
- 37.: hub fitting
- 38.: wire guide port
- 39.: proximal end
- 41.: balloon
- 42.: closed distal end
- 43.: proximal balloon bond
- 44.: outer surface
- 45.: length
- 50.: column strength stiffener
- 51.: proximal end
- 52.: balloon support segment
- 53.: distal end
- 60.: wire guide
- 61.: distal end
- 62.: segment
- 63.: curved distal segment
- 65.: wire guide
- 67.: sheath
- 70.: stent
- 71.: interior surface
- 72.: covered stent graft
- 73.: distal end
- 80.: fenestrated stent graft
- 81.: fenestration
- 82.: branched covered stent
- 85.: fenestration
- 165.: wire guide
- 167.: sheath
- 172.: covered stent graft

## Claims

1. A stent delivery balloon catheter assembly comprising:
a catheter that defines exactly one or two lumens, which includes an inflation lumen that extends from an inflation port at a proximal end to open through a distal end of the catheter, and defining a wire guide channel that terminates at a wire guide exit located a distance from the distal end;
a balloon with a closed distal end attached to the catheter distally of the wire guide exit, and extending distally beyond the distal end of the catheter, and the balloon being in fluid communication with the inflation lumen.
a column strength stiffener with a proximal end attached to the catheter distally of the wire guide exit, a balloon support segment extending distally away from the distal end of the catheter within the balloon, and a distal end of the column strength stiffener being attached to the closed distal end of the balloon.

2. The stent delivery balloon catheter assembly of claim 1 wherein wire guide channel is a wire guide lumen of the exactly two lumens.

3. The stent delivery balloon catheter assembly of any of claim 1 wherein the catheter defines exactly one lumen, which is the inflation lumen; and
the wire guide channel opens in a direction away from a centerline of the catheter between two channel edges that are out of contact with each other.

4. The stent delivery balloon catheter assembly of any of claims 1-3 including a wire guide received in the wire guide channel and having a distal end positioned at, or proximally displaced from, the wire guide exit.

5. The stent delivery balloon catheter assembly of claim 3 or 4 including a sheath; wherein the balloon is slidably positioned within the sheath.

6. The stent delivery balloon catheter assembly of any of claims 1-4 wherein the wire guide channel includes a wire guide entrance located a distance along a catheter centerline from the proximal end of the catheter.

7. The stent delivery balloon catheter assembly of any of claims 1-5 wherein the wire guide channel is sized to slidably receive a 0.035 inch diameter wire guide; and
the wire guide exit is at least one centimeter away from a proximal balloon bond to the catheter.

8. The stent delivery balloon catheter assembly of any of claims 1-6 including a hub fitting attached to the proximal end of the catheter, and the hub fitting including the inflation port and a wire guide port that is a proximal end of the wire guide channel.

9. A stent delivery system comprising:
a sheath;
a wire guide;
a stent;
a stent delivery balloon catheter that includes a catheter that defines an inflation lumen, and the catheter defining a wire guide channel that terminates at a wire guide exit located a distance from a distal end of the catheter; and a balloon with a closed distal end attached to the catheter distally of the wire guide exit, and extending distally beyond the distal end of the catheter, and the balloon being in fluid communication with the inflation lumen; and a column strength stiffener with a proximal end attached to the catheter distally of the wire guide exit, a balloon support segment extending distally away from the distal end of the catheter within the balloon, and a distal end of the column strength stiffener being attached to the closed distal end of the balloon;
the system has a first configuration **characterized by** the stent being crimped about the balloon, which is deflated and positioned inside the sheath, and a distal end of the wire guide is positioned at, or proximally displaced from, the wire guide exit and within the wire guide channel; and
the system has a second configuration **characterized by** the stent and the balloon being outside of the sheath, and the distal end of the wire guide is positioned between an outer surface of the balloon, which is deflated, and an interior surface of the stent, which is expanded, and a segment of the wire guide is positioned in the wire guide channel.

10. The system of claim 9 wherein the stent at least one of:
(i) is a covered stent graft; and
(ii) extends through a fenestration of a fenestrated stent graft.

11. The system of any of claims 9-10 wherein the wire guide has a 0.035 inch diameter and a curved distal segment.

12. The system of any of claims 9-11 including a third configuration **characterized by** the wire guide being pinched between the outer surface of the balloon, which is inflated, and less than a full length of the interior surface of the stent.

13. The system of any of claims 9-12 wherein the balloon is positioned in a fenestration of a fenestrated stent graft in the third configuration.

14. A method of operating a stent delivery system comprising the steps of:
moving a distal end of a sheath from within a fenestrated stent graft and out through a fenestration of the fenestrated stent graft;
moving a stent delivery balloon catheter through the sheath to a position at which a balloon of the stent delivery balloon catheter is within the sheath, and most or all of a length of the balloon is outside of the fenestrated stent graft;
positioning a wire guide in a wire guide channel of the delivery balloon catheter so that a distal end of the wire guide is positioned at, or proximally displaced from, a wire guide exit located proximal to the balloon;
moving the sheath in a proximal direction to uncover a covered stent graft that is crimped about the balloon, while maintaining a position of the stent delivery balloon catheter;
expanding the covered stent graft by inflating the balloon;
deflating the balloon;
advancing the distal end of the wire guide out through the wire guide exit, along an outer surface of the balloon, into the expanded covered stent graft and out through a distal end of the covered stent graft; and
withdrawing the stent delivery balloon catheter in a proximal direction along the wire guide, while maintaining the wire guide in place.

15. The method of claim 14 wherein the positioning step includes at least one of:
(i) moving the wire guide along the wire guide channel after the stent delivery balloon catheter is at the position at which a balloon of the stent delivery balloon catheter is within the sheath, and most or all of a length of the balloon is outside of the fenestrated stent graft; and
(ii) moving the stent delivery balloon catheter with the wire guide positioned in the wire guide channel through the sheath to the position at which a balloon of the stent delivery balloon catheter is within the sheath, and most or all of a length of the balloon is outside of the fenestrated stent graft.

16. The method of any of claims 14-15 including the steps of:
moving the balloon after the deflating step; and
re-inflating the balloon within the fenestration.
